(19) Europäisches Patentamt
European Patent Office
Office européen des brevets

(11) Veröffentlichungsnummer : **0 002 802**
**B1**

(12) **EUROPÄISCHE PATENTSCHRIFT**

(45) Veröffentlichungstag der Patentschrift : **02.12.81**

(51) Int. Cl.³ : **C 07 C 85/11**, C 07 C 87/60

(21) Anmeldenummer : **78101796.7**

(22) Anmeldetag : **21.12.78**

(54) **Verfahren zur Herstellung von Halogenanilinen.**

(30) Priorität : **24.12.77 DE 2758111**

(43) Veröffentlichungstag der Anmeldung : **11.07.79 (Patentblatt 79/14)**

(45) Bekanntmachung des Hinweises auf die Patenterteilung : **02.12.81 Patentblatt 81/48**

(84) Benannte Vertragsstaaten : **BE CH DE FR GB IT**

(56) Entgegenhaltungen :
**DE - A - 2 032 565**
**DE - A - 2 240 849**
**DE - A - 2 503 187**
**DE - A - 2 615 079**

(73) Patentinhaber : **BASF Aktiengesellschaft**
**Carl-Bosch-Strasse 38**
**D-6700 Ludwigshafen (DE)**

(72) Erfinder : **Jersak, Ulrich, Dr.**
**Dubliner Strasse 21**
**D-6700 Ludwigshafen (DE)**
Erfinder : **Scheuermann, Horst, Dr.**
**Bexbacher Strasse 41**
**D-6700 Ludwigshafen (DE)**
Erfinder : **Gaeng, Manfred, Dr.**
**An der Tuchbleiche 11**
**D-6712 Bobenheim-Roxheim 2 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Imprimerie Jouve, 17, rue du Louvre, 75001 Paris, France

Verfahren zur Herstellung von Halogenanilinen

Die Erfindung betrifft ein neues Verfahren zur Herstellung von Halogenanilinen durch katalytische Hydrierung von Halogennitrobenzolen unter bestimmten Bedingungen bezüglich Lösungsmittelmenge und pH in Gegenwart von ein oder mehreren Metallen mit einer Ordnungszahl zwischen 24 und 29.

Nach Houben-Weyl, Methoden der Organischen Chemie, Band 11/1, Seiten 360 bis 382, ist die Reduktion aromatischer Nitroverbindungen die älteste und immer noch wichtigste Methode zur Herstellung primärer aromatische Amine. Wasser und niedere Alkohole werden als Lösungsmittel verwendet (Seiten 366 und 367). 3-Nitrochlorbenzol kann in Mengen von 30 Gramm in Gegenwart von 100 Kubikzentimetern Alkohol und Raneynickel bei Zimmertemperatur und einem Druck von 2 bis 3 atü Wasserstoff zu 3-Chloranilin reduziert werden (Seite 370). Eine entsprechende Reaktion wird für das 4-Nitrochlorbenzol (Seite 376) angegeben. Diese Reduktion mit katalytisch erregtem Wasserstoff im Labormaßstab kann aber industriell nicht zur Herstellung von Chlor- bzw. Dichloranilinen dienen, da konkurrierend zur Reduktion der Nitrogruppe unerwünschte Abspaltung der Chloratome eintritt und sich in größerem Maßstab die Ausbeuten verschlechtern (US-Patentschrift 3 145 231, Spate 1). In der Fachliteratur sind verschiedene Zusätze oder Vorbehandlungen der Hydrierkatalysatoren angegeben, um die Dehalogenierung während der Hydrierung der Nitrogruppe weitgehend zu unterbinden. So wird in der US-Patentschrift 3 941 717 die Vorbehandlung eines Platin- oder Palladium-Katalysators zuerst mit einem Sulfoxid und anschließend mit Hydrazin für die Hydrierung von Chlornitrobenzolen, z.B. 3,5-Dichlornitrobenzol, angegeben. In der US-Patentschrift 3 145 231 wird die Hydrierung von 3,5-Dichlornitrobenzol mit einem Platin-Katalysator unter Zusatz einer cycloaliphatischen Stickstoffbase als Dehalogenierungsinhibitor beschrieben.

Die Hydrierung von 3-Chlornitrobenzol soll nach der Lehre der US-Patentschrift 3 474 144 ohne Dehalogenierung gelingen, wenn dem Platin-Katalysator (Kohleträger) ein Phosphit zugesetzt wird. Die Verwendung eines Katalysatorsystems mit einem Platin-Katalysator, einer Stickstoff-Base, zweiwertigem Nickel und dreiwertigem Kobalt zur Hydrierung von Chlornitrobenzolen wie 3,5-Dichlornitrobenzol wird in dem US-Patent 3 546 297 beschrieben. Auch Nickelkatalysatoren sollen nur zusammen mit Dehalogenierungsinhibitoren zur Herstellung von halogenierten, aromatischen Aminen aus den entsprechenden Nitroaromaten eingesetzt werden können; so werden in der US-Patentschrift 3 067 253 Zusätze von Calciumhydroxid oder Magnesiumhydroxid bzw. Natrium- oder Calciumcarbonaten bzw. entsprechenden Acetaten beschrieben. In der DOS 1 643 379 wird der Zusatz von Rhodaniden zum Nickelkatalysator angegeben. Die deutsche Offenlegungsschrift 2 240 849 lehrt die Verwendung eines pH von 6 bis 7,5 zu Beginn der Reaktion und Beibehaltung dieses pH-Intervalls während der gesamten Hydrierung in Gegenwart von Nickelkatalysatoren.

Alle diese Verfahren sind im Hinblick auf einfachen und wirtschaftlichen Betrieb, Lösungsmittelverluste, Ausbeute und Reinheit des Ausgangsstoffes unbefriedigend.

Es wurde non gefunden, daß man Chloraniline der Formel

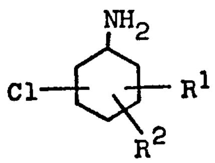

(I)

worin $R^1$ und $R^2$ gleich oder verschieden sein können und jeweils ein Wasserstoffatom oder einen aliphatischen Rest bedeuten, $R^1$ auch ein Chloratom bezeichnet, durch katalytische Hydrierung von Halogennitrobenzolen vorteilhaft erhält, wenn man Chlornitrobenzol der Formel

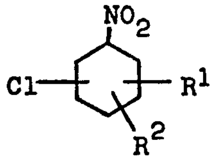

(II)

worin $R^1$ und $R^2$ die vorgenannte Bedeutung besitzen, unter Verwendung von weniger els 20 Gewichtsprozent unter den Reaktionsbedingungen inertem Lösungsmittel, bezogen auf Ausgangsstoff II, in Gegenwart von ein oder mehreren Metallen mit einer Ordnungszahl zwischen 24 und 29 als Hydrierkatalysator hydriert, wobei man die Hydrierung bei einem pH von 2 bis 4,5 beginnt.

Die Umsetzung kann für den Fall der Verwendung von 3-Chlornitrobenzol durch die folgenden Formeln wiedergegeben werden:

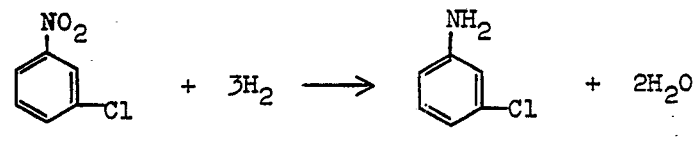

Im Hinblick auf den Stand der Technik liefert das Verfahren nach der Erfindung auf einfacherem und wirtschaftlicherem Wege Chloraniline in teilweise besserer Ausbeute und Reinheit. Lösungsmittel werden in geringerer Menge angewendet, man hydriert sogar vorteilhafter in Abwesenheit von Lösungsmitteln, was geringere Lösungsmittelverluste und einfacheren und sichereren Betrieb mit sich bringt. Alle diese vorteilhaften Eigenschaften des erfindungsgemäßen Verfahrens sind im Hinblick auf die bekannten Verfahren überraschend.

Bevorzugte Ausgangsstoffe II und dementsprechend bevorzugte Endstoffe I sind solche, in deren Formel $R^1$ und $R^2$ gleich oder verschieden sein können und jeweils ein Wasserstoffatom oder einen Alkylrest mit 1 bis 6 Kohlenstoffatomen bedeuten, $R^1$ auch ein Chloratom bezeichnet. Die vorgenannten Reste können noch durch unter den Reaktionsbedingungen inerte Gruppen, z.B. Alkylgruppen oder Alkoxygruppen mit jeweils 1 bis 4 Kohlenstoffatomen, substituiert sein.

Es kommen z.B. folgende Ausgangsstoffe II in Betracht : Methyl-, Äthyl-, Propyl-, Isopropyl-, Butyl-, Isobutyl-, tert.-Butyl-, sek.-Butyl-(4)-2-chlornitrobenzol und entsprechend in 3-Stellung, 5-Stellung und 6-Stellung durch vorgenannte Alkylgruppen substituierte 2-Chlornitrobenzole ; homologe, in 3-oder 4-Stellung durch Chlor und entsprechend in 4- oder 3-Stellung bzw. 2-, 5- oder 6-Stellung durch vorgenannte Alkylgruppen substituierte Chlornitrobenzole ; in 2-, 3-, 4-, 5- und/oder 6-Stellung zweimal durch Chloratome und einmal durch eine der vorgenannten Alkylgruppen substituierte Dichlornitrobenzole ; vorteilhaft unsubstituiertes 2- oder 4-Monochlornitrobenzol, 2,4-, 2,3-, 2,5-, 2,6- oder 3,4-Dichlorbenzol, bevorzugt 3-Chlornitrobenzol oder 3,5-Dichlornitrobenzol.

Die Umsetzung wird in der Regel bei einer Temperatur von 20 °C bis 200 °C, vorzugsweise von 50 bis 100 °C, drucklos oder zweckmäßig unter Druck, kontinuierlich oder dikkontinuierlich durchgeführt. Zu Beginn der Hydrierung ist die Temperatur zweckmäßig so hoch, daß das Ausgangsgemisch als Schmelze vorliegt. Da wegen der im Vergleich zu den entsprechenden Nitroaromaten tiefer liegenden Erstarrungspunkte der Chloraniline die Erstarrungstemperatur der Schmelze mit fortschreitendem Umsatz absinkt, ist der Temperaturbereich zweckmäßig 20 °C bis 180 °C für die Hydrierung von 3-Chlornitrobenzol und 52 °C bis 180 °C für die Herstellung von 3,5-Dichloranilin, bevorzugt 55 bis 100 °C. Hydriert wird vorteilhaft bei einem Druck von 1 bis 100 bar, vorzugsweise 1 bis 50 bar, insbesondere 1 bis 10 bar.

Die Umsetzung kann mit unter den Reaktionsbedingungen inerten Lösungsmitteln in einer Menge von weniger als 20 Gewichtsprozent, zweckmäßig 0 bis 15 Gewichtsprozent, vorteilhaft 0 bis 10 Gewichtsprozent Lösungsmittel, bezogen auf Ausgangsstoff II, durchgeführt werden. Zweckmäßig sind als Lösungsmittel Alkohole oder vorteilhaft Wasser. Als Alkohole kommen z.B. Methanol, Äthanol, n- und i-Propanol, n-Butanol, Butanol-2, Isobutanol, Äthylenglykol, Diäthylenglykol, Methyläthylenglykol, Benzylalkohol, n-Pentanol, Phenyläthanol, Neopentylglykol, p-Methylbenzylalkohol, p-Äthoxybenzylalkohol, 1,3-Propylenglykol, 1,4-Butandiol, 1,2-Propylenglykol, Triäthylenglykol, Diäthylenglykol-mono-n-butyläther ; oder entsprechende Gemische in Frage. Bevorzugt sind Methanol, Äthanol, Isopropanol, Methyläthylenglykol, n-Propanol, Isobutanol.

Als Lösungsmittel kommen gegebenenfalls auch Ketone, z.B. Aceton ; bzw. Tetrahydrofuran, Toluol, Xylol, Chlorbenzol oder Dimethylformamid in Betracht. Falls es zur Abführung der Reaktionswärme, insbesondere bei kontinuierlicher Fahrweise, zweckmäßig ist, kann das Ausgangsgemisch auch vorteilhaft mit 3-Chloranilin, zweckmäßig bei der Hydrierung von 3-Chlornitrobenzol, bzw. mit 3,5-Dichloranilin, zweckmäßig bei der Hydrierung von 3,5-Dichlornitrobenzol, verdünnt werden, vorzugsweise in einer Menge von 5 bis 50 Gewichtsprozent vorgenanntem Mono- oder Dichloranilin, bezogen auf Ausgangsstoff II.

Bevorzugt wird der Ausgangsstoff II in Abwesenheit von Lösungsmitteln in Gestalt einer Schmelze hydriert.

Das Ausgangsgemisch wird vor Beginn der Umsetzung durch Zusätze, zweckmäßig Säuren, so eingestellt, daß man die Umsetzung bei einem pH von 2 bis 4,5, vorteilhaft 2,5 bis 4 beginnt. Während der Umsetzung steigt das pH, im allgemeinen liegt der Endwert des pH bei 5 bis 6,5, insbesondere bei 5 bis 6. Während der Umsetzung wird in der Regel keine Säure nachgesetzt. Es kommen im allgemeinen anorganische Säuren in Betracht. Anstelle einbasischer Säuren können auch äquivalente Mengen mehrbasischer Säuren oder saure Salze, insbesondere Natrium- und Kaliumsalze, zur Anwendung gelangen. Beispielsweise sind folgende Säuren geeignet : Chlorwasserstoff, Bromwasserstoff, Jodwasserstoff, Schwefelsäure, Phosphorsäure, Salpetersäure ; Bor enthaltende Säuren wie Borsäure, Borfluorwasserstoffsaure ; oder entsprechende Gemische. Die Säuren können in konzentrierter Form, im Gemisch miteinander und/oder mit einem Lösungsmittel, insbesondere Wasser, angewendet werden. Bevorzugt sind Schwefelsäure, Salzsäure, Phosphorsäure, $KHSO_4$, $KH_2PO_4$, $NaHSO_4$, $NaH_2PO_4$.

Die Umsetzung wird in Gegenwart von Wasserstoff in stöchiometrischer Menge oder im Überschuß, im allgemeinen in einer Menge von 3 bis 25, vorzugsweise von 3 bis 50 Molprozent, bezogen auf Ausgangsstoff II, durchgeführt. Man kann den Wasserstoff kontinuierlich oder diskontinuierlich der

Reaktion zuführen und/oder den Katalysator selbst nach einer bestimmten Reaktionszeit wieder frisch mit Wasserstoff beladen. Die Umsetzung wird mit Wasserstoff in Gegenwart eines Hydrierkatalysators durchgeführt. Als Hydrierkatalysator verwendet man ein oder mehrere Metalle mit einer Ordnungszahl zwischen 24 und 29, in der Regel Kobalt- oder Nickelkatalysatoren, z.B. entsprechende Sinterkatalysatoren, die bis zu 30 Gewichtsprozent Mangan und/oder Eisen enthalten können ; vorzugsweise gelangen Raney-Nickel und Raney-Kobalt zur Verwendung. Der Hydrierkatalysator wird in der Regel in Mengen von 0,05 bis 50 Gewischtsprozent, zweckmäßig in einer Menge von 0,05 bis 15 Gewichtsprozent, vorzugsweise 0,1 bis 5 Gewichtsprozent, bezogen auf Ausgangsstoff II, zugesetzt. In der Regel werden dem Reaktionsgemisch am Anfang und im Verlauf der Umsetzung solche Mengen an Wasserstoff zugeführt, daß sich bei der Umsetzungstemperatur stets ein entsprechender Reaktionsdruck einstellt. Zur entsprechenden Druckeinstellung können auch inerte Gase, wie Stickstoff, neben Wasserstoff verwendet werden.

Die Umsetzung kann wie folgt durchgeführt werden : Man gibt den Ausgangsstoff II, gegebenenfalls zusammen mit dem Lösungsmittel, in einen Reaktor, setzt den Hydrierkatalysator zu und spült den Reaktionsraum mit Stickstoff. Dann wird Wasserstoff bis zu vorgenanntem Reaktionsdruck eingepreßt. Nun wir das Reaktionsgemisch auf vorgenannte Temperatur gebracht und so lange bei dieser Temperatur unter Einleitung von weiterem Wasserstoff gehalten, bis kein Wasserstoff mehr durch die Reaktion verbraucht wird ; im allgemeinen beträgt diese Reaktionszeit zwischen 15 und 100 Stunden. Nun wird das Reaktionsgemisch abgekühlt und filtriert. Aus dem Filtrat wird der Endstoff nach den üblichen Methoden, z.B. durch Eindampfen des Filtrats bzw. Abtrennung der organischen Phase, abgetrennt. Bei der Hydrierung einer lösungsmittelfreien Schmelze ist die folgende Aufarbeitungsweise vorteilhaft : Nach Beendigung der Hydrierung läßt man das Gemisch kurze Zeit, z.B. 60 Minuten, stehen, trennt dann die obere aus Reaktionswasser bestehende Phase ab, filtriert die organische Phase zur Entfernung des Katalysators und erhält so den Endstoff in sehr guter Reinheit nahezu wasserfrei.

In einer vorteilhaften Ausführungsform verwendet man rohen Ausgangsstoff II, wie er aus dem Reaktionsgemisch seiner Herstellung, zweckmäßig nach der in der deutschen Patentschrift 2 555 736 beschriebenen Arbeitsweise durch Umsetzung von Halogennitroanilinen mit Alkanolen und Nitrosierungsmitteln in Gegenwart von Wasser und Säure bei mindestens 35 °C, anfällt. Die Reaktion kann z.B. wie folgt durchgeführt werden : Ein Gemisch von Halogennitroanilin, Alkohol, Nitrosierungsmittel, Säure und Wasser wird während 1,5 bis 5 Stunden bei der Reaktionstemperatur gehalten. Zweckmäßig läßt man das Nitrosierungsmittel, z.B. die wäßrige Natriumnitritlösung oder den Salpetrigsäureester zum Gemisch der Reaktionspartner zulaufen. Der Zulauf kann in einem weiten Bereich beliebig schnell erfolgen. Das Reaktionsende fällt meist mit dem Ende des Zulaufs des Nitrosierungsmittels zusammen. Aus dem Reaktionsgemisch wird zweckmäßig der Endstoff durch Phasentrennung bei 70 °C abgetrennt und in vorgenannter Weise als Ausgangsstoff II des erfindungsgemäßen Verfahrens hydriert. Vorteilhaft setzt man gerade für die Herstellung von 3,5-Dichloranilin ein nach der deutschen Patentschrift 2 555 736 synthetisiertes und in geschmolzener Form aus dem Reaktionsgemisch abgetrenntes 3,5-Dichlornitrobenzol ein, das in der Regel den erfindungsgemäßen pH-Wert zeigt ; falls der pH-Wert $< 2$ beträgt, kann abgestumpft werden, z.B. mit Natriumacetat, oder, falls er $< 4,5$ ist, wird mit vorgenannten Säuren auf den erfindungsgemäße pH-Wert eingestellt.

Die nach dem Verfahren der Erfindung herstellbaren neuen Verbindungen sind wertvolle Ausgangsstoffe für die Herstellung von Farbstoffen und Schädlingsbekämpfungsmitteln. Bezüglich der Verwendung wird auf vorgenannte Veröffentlichungen und Ullmanns Encyklopädie der technischen Chemie, Band 3, Seite 651, verwiesen.

In den nachfolgenden Beispielen bedeuten Teile Gewichtsteile.

## Beispiel 1

In einem Rührkessel werden 30 000 Teile 3,5-Dichlornitrobenzol geschmolzen. 50 Teile 50-gewichtsprozentige Schwefelsäure und dann 750 Teilen Raney-Nickel werden hinzugefügt. Das Gemisch wird bei 65 bis 70 °C mit Wasserstoff von einem Druck von 5 bar hydriert. Nach 40 Stunden wird kein Wasserstoff mehr aufgenommen. Das Reaktionsgemisch hatte zu Beginn der Reaktion ein pH von 3,5, bei Ende der Reaktion von 5,8. Insgesamt werden 940 Teile $H_2$ verbraucht. Nach dem Abfiltrieren des Katalysators werden die gebildeten Phasen getrennt. Die spezifisch schwerere Phase besteht aus 24 807 Teilen 3,5-Dichloranilin (98 % der Theorie) vom Schmelzpunkt 50 bis 51 °C und 150 Teilen Wasser.

## Beispiel 2

Man trägt 207 Teile 2,6-Dichlor-4-nitro-anilin in 180 Teile Isopropanol und 300 Teile Wasser ein und versetzt das Gemisch dann mit 200 Teilen konzentrierter Schwefelsäure (98 Gewichtsprozent). Bei 50 °C läßt man die Lösung von 125 Teilen $NaNO_2$ in 175 Teilen Wasser zulaufen, wobei sich Stickstoff entwickelt. Dann fügt man 400 Teile Wasser hinzu und läßt 60 Minuten bei 65 °C stehen. Dann wird die spezifisch schwerere Phase abgetrennt und zu dieser Phase wird bei 65 °C 1 Teil Natriumacetat zugegeben, wobei sich im Gemisch ein $pH_4$ einstellt. Dann wird analog Beispiel 1 hydriert. Insgesamt werden 6 Teile $H_2$ verbraucht. Das Reaktionsgemisch hatte zu Beginn der Reaktion ein pH von 4, bei

Ende der Reaktion von 6. Man erhält 157 Teile 3,5-Dichloranilin (97 % der Theorie) vom Schmelzpunkt 49 bis 51 °C.

Beispiel 3

30 000 Teile 3,5-Dichlornitrobenzol, 3 000 Teile Wasser und 3 000 Teile Isopropanol werden auf 80 °C erhitzt. Nach Zugabe von 30 Teilen konzentrierter Schwefelsäure (98 Gewichtsprozent) werden 750 Teile Raney-Nickel hinzugefügt. Dann wird das Gemisch bei 80 °C mit Wasserstoff von 2 bar Druck hydriert. Nach 40 Stunden wird kein Wasserstoff mehr aufgenommen. Insgesamt werden 940 Teile $H_2$ verbraucht. Das Reaktionsgemisch hatte zu Beginn der Reaktion ein pH von 3,2, bei Ende der Reaktion von 6. Lösungsmittel und Wasser werden abdestilliert; dann wir den Raney-Nickel abfiltriert. Man erhält 23 794 Teile 3,5-Dichloranilin (94 % der Theorie) vom Schmelzpunkt 48 bis 50 °C.

Beispiel 4

Zu 157,5 Teilen 3-Chlornitrobenzol werden 0,15 Teile Natriumhydrogensulfat in 20 Teilen Wasser hinzugefügt. Der pH liegt dann bei 3. Es wird auf 65 °C aufgeheizt und analog Beispiel 1 hydriert. Insgesamt werden 6 Teile $H_2$ verbraucht. Das Gemisch hat am Ende einen pH von 6,2. Man erhält 121 Teile 3-Chloranilin (95 % der Theorie) mit Siedepunkt 227 bis 230 °C.

**Anspruch**

Verfahren zur Herstellung von Chloranilinen der Formel

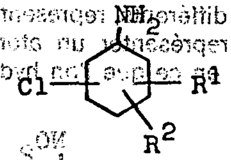

(I)

worin $R^1$ und $R^2$ gleich oder verschieden sein können und jeweils ein Wasserstoffatom oder einen aliphatischen Rest bedeuten, $R^1$ auch ein Chloratom bezeichnet, durch katalytische Hydrierung von Halogenitrobenzolen, dadurch gekennzeichnet, daß man Chlornitrobenzole der Formel

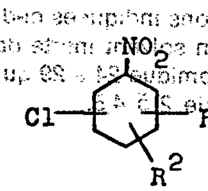

(II)

worin $R^1$ und $R^2$ die vorgenannte Bedeutung besitzen, unter Verwendung von weniger als 20 Gewichtsprozent unter den Reaktionsbedingungen inerten Lösungsmittels, bezogen auf Ausgangsstoff II, in Gegenwart von ein oder mehreren Metallen mit einer Ordnungszahl zwischen 24 und 29 als Hydrierkatalysator Hydriert, wobei man die Hydrierung bei einem pH von 2 bis 4,5 beginnt.

**Claim**

Process for the preparation of chloroanilines of the formula

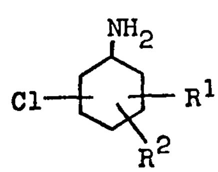

(I)

where $R^1$ and $R^2$ can be identical or different and each is a hydrogen atom or an aliphatic radical, and $R^1$ can also be a chlorine atom, by catalytic hydrogenation of halonitrobenzenes, characterised in that chloronitrobenzenes of the formula

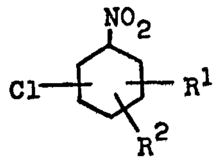

(II)

where $R^1$ and $R^2$ have the above meaning, are hydrogenated, using less than 20 per cent by weight, based on starting material II, of a solvent which is inert under the reaction conditions, in the presence of one or more metals of atomic order number from 24 to 29 as a hydrogenation catalyst, the hydrogenation being started at a pH of from 2 to 4.5.

**Revendication**

Procédé de préparation de chloranilines de formule

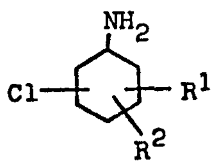

(I)

dans laquelle $R^1$ et $R^2$, identiques ou différents, représentent chacun un atome d'hydrogène ou un reste aliphatique, $R^1$ pouvant également représenter un atome de chlore, par hydrogénation catalytique d'halogénonitrobenzènes, caractérisé en ce que l'on hydrogène des chloronitrobenzènes de formule

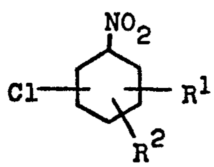

(II)

dans laquelle $R^1$ et $R^2$ ont les significations indiquées ci-dessus, en utilisant moins de 20 % en poids, par rapport au composant de départ II, d'un solvant inerte dans les conditions de la réaction, en présence d'un ou plusieurs métaux de nombre atomique 24 à 29 qui servent de catalyseurs d'hydrogénation, et en commençant l'hydrogénation à un pH de 2 à 4,5.